# EUROPEAN PATENT SPECIFICATION

(11) **EP 0 689 600 B1**
(45) Date of publication and mention of the grant of the patent: **23.05.2001**
(21) Application number: 94910816.1
(22) Date of filing: 02.03.1994
(51) Int. Cl.: C12N 15/62, C07K 14/52, C12N 15/28, A61K 38/19, A61K 39/395

(54) **PROCESS TO INDUCE THE DEATH OF TUMOR CELLS**
VERFAHREN ZUR INDUKTION DES TODES VON TUMORZELLEN
PROCEDE POUR L'INDUCTION DE LA MORT DE CELLULES TUMORALES

(30) Priority: 11.03.1993 US 29752
(43) Date of publication of application: 03.01.1996
(73) Proprietor: Genentech, Inc., South San Francisco, CA 94080-4990 (US)
(72) Inventor: WONG, Grace H.W., South San Francisco, CA 94080 (US)
(74) Representative: Armitage, Ian Michael
(86) International application number: US9402280
(87) International publication number: WO9420625

(56) References cited:
- EP-A- 0 510 691
- NAUNYN-SCHMIEDEBERG'S ARCHIVES OF PHARMACOLOGY vol. 344, no. SUP2 , 1991 , SPRINGER-VERLAG page R112 HOLTMANN H;KOENIG M;WALLACH D;YONEHARA S;RESCH K; 'COMPARISON OF THE INDUCTION OF CELL DEATH BY TUMOR NECROSIS FACTOR AND ANTI-FAS MONOCLONAL ANTIBODIES'
- INTERNATIONAL IMMUNOLOGY vol. 3, no. 4 , 1991 pages 343 - 351 HASHIMOTO S;ISHII A;YONEHARA S; 'The E1b oncogene of adenovirus confers cellular resistance to cytotoxicity of tumor necrosis factor and monoclonal anti-Fas antibody.'
- PATENT ABSTRACTS OF JAPAN vol. 14, no. 555 (C-0786) 10 December 1990 & JP,A,02 237 935 (BIO KAGAKU KENKYUSHO: KK) 20 September 1990

## Description

### BACKGROUND OF THE INVENTION

### I. Field of the Invention

The invention relates to method and means for cell lysis or inhibition of unwanted cell growth. More particularly, the invention concerns the killing or inhibition of growth of tumor and virus infected cells expressing both the Fas antigen and the type 1 TNF receptor (TNF-R1).

### II. Description of Background and Related Art

It has been known for decades that certain cells are programmed to die at certain points of development - a process termed "apoptosis". Cells dying by apoptosis are readily identifiable by a set of characteristic morphological and biochemical changes, such as condensation of cytoplasm, loss of plasma membrane microvilli, segmentation of the nucleus, and extensive degradation of chromosomal_DNA into oligomers of about 180 bp. Apoptosis is essential in many physiological processes including embryonic development and clonal selection in the immune system [Itoh *et al.,* Cell 66, 233-243 (1991)], and can be triggered by various stimuli such as glucocorticoid hormones for immature thymocytes, and withdrawal of certain growth factors [Watanabe-Fukunaga *et al.,* Nature 356, 314-317 (1992)]. Overexpression of bcl-2 P53 or c-myc appears to modulate apoptosis. Apoptosis has been observed as instrumental in tumor cell regression, and it was proposed that tumor cells can be destroyed by artificial induction of apoptosis. Indeed, cytotoxic drugs such as, tumor necrosis factor-alpha (TNF-α) and tumor necrosis factor-beta (TNF-β, lymphotoxin), and radiation were shown to induce apoptotic death in susceptible tumor cells [Schmid et *al.,* Proc. Natl. Acad. Sci. USA 83, 1881 (1986); Dealtry *et al.,* Eur. J. Immunol. 17, 689 (1987);

The first step in the induction of the various cellular responses mediated by TNF-α or TNF-β is their binding to specific cell surface receptors. Two distinct TNF receptors of approximately 55-kDa (TNF-R1) and 75-kDa (TNF-R2) have been identified [Hohman, H.P. et al., J. Biol. Chem. 264, 14927-14934 (1989); Brockhaus, M. et al., Proc. Natl. Acad. Sci. USA 87, 3127-3131 (1990)], and human and mouse cDNAs corresponding to both receptor types have been isolated and characterized [Loetscher, H. et al., Cell 61, 351 (1990); Schall, T.J. et al., Cell 61, 361 (1990) (11-15); Smith, C.A. et al., Science 248, 1019 (1990); Lewis, M. *et al*., Proc. Natl. Acad. Sci. USA 88, 2830-2834 (1991); Goodwin, R.G. *et al.,* Mol. Cell. Biol. 11, 3020-3026 (1991)].

Although not yet systematically investigated, the majority of cell types and tissues appear to express both TNF receptors. In attempting to understand the individual roles of the two TNF receptors, several groups have generated poly- and monoclonal antibodies (mAbs) that are specific for either TNF-R1 or TNF-R2.

It has been observed that both polyclonal and monoclonal antibodies directed against TNF-R1 can act as specific agonists for this receptor and elicit several TNF activities such as cytotoxicity, fibroblast proliferation, resistance to chlamidiae, synthesis of prostaglandin E₂ and antiviral activity [Engelman, H. *et al.,* J. Biol. Chem. 265, 14497-14504 (1990); Espevik, T. *et al.,* J. Exp. Med. 171, 415-426 (1990); Shalaby, M.R. *et al.,* J. Exp. Med. 172, 1517-1520 (1990); Wong G. *et al.,* J. Immunol. 149, 3350-3353 (1992)]. Agonist antibodies to TNF-R1 with antiviral activity are disclosed in Wong *et al.,* J. Immunol. 149, 3350-3353 (1992). In addition, polyclonal antibodies to both murine TNF-R1 and TNF-R2 have been developed, and each shown to behave as specific receptor agonists and induce a subset of murine TNF activities. While the murine TNF-R1 was shown to be responsible for signaling cytotoxicity and the induction of several genes, the murine TNF-R2 was shown to be capable of signaling proliferation of primary thymocytes and a cytotoxic T cell line [Tartaglia, L.A. et al., Proc. Natl. Acad. Sci. USA 88, 9292-9296 (1991)].

The Fas antigen (also referred to as APO-1 or Fas receptor) is a cell surface molecule that belongs to the tumor necrosis factor (TNF) receptor - nerve growth factor (NGF) receptor superfamily, and has recently been implicated in the negative selection of autoreactive T cells in the thymus. Fas receptor can signal an apoptotic cell death similar to that signaled by the 55-kDa TNF-R1 [Itoh *et al.,* Cell 66, 233-243 (1991)]. Agonist mouse monoclonal antibodies specifically binding to the Fas antigen have been reported to exhibit cell-killing activity that is indistiguishable from that of TNF-α, and it was suggested that the cytolytic activity of TNF-α is mediated by Fas antigen associated with TNF-R1. Expression of the Fas antigen was reported to be down-regulated along with that of TNF-R1 when cells were treated with either TNF-a or anti-Fas mouse monoclonal antibody. The cytolytic activity of TNF-a and anti-Fas antibody was augmented by cotreatment of cells with interferon-γ [Yonehara *et al*., J. Exp. Med. 169, 1747-1756 (1989)]. These findings were interpreted in the literature as predicting that cell lines that co-express both Fas and TNF-R1 receptors mediate cell killing through common signaling pathways. Both TNF and anti-Fas monoclonal antibodies were shown to be cytocydal to human immunodeficiency virus (HIV)-infected cells, however, TNF but not anti Fas antibody augments HIV replication [Kobayashi *et al.,* Proc. Natl. Acad. Sci. USA 87, 9620-9624 (1990)].

### SUMMARY OF THE INVENTION

The present invention is based on the unexpected finding that Fas antigen and TNF-R1 signal cell killing through distinct signaling pathways.

By screening a large number of both TNF-α resistant and TNF-α sensitive cell lines for killing by agonist anti-Fas antibodies it has been observed that some tumor cells co-expressing both functional Fas and TNF-R1 receptors could be killed by agonist antibodies specifically binding the Fas antigen but not by TNF-α or agonist antibodies specifically binding TNF-R1. Other cell lines that were highly sensitive to TNF-α and anti-TNF-R1 antibodies showed no sensitivity to agonist anti-Fas antibodies. For example, Fas agonists did not kill normal fibroblast or lymphoid cells even when they expressed very high levels of Fas antigen.

It has further been observed that a combination of TNF-R1 agonists and Fas agonists exhibit a synergistic interaction in their ability to kill tumor cells. Pretreatment with TNF-α, agonist antibodies specifically binding TNF-R1, IL-1 (probably through induction of protective proteins), or cycloheximide (may inhibit labile killing protein synthesis) was observed to protect against subsequent killing by TNF-a but not by anti-Fas agonist antibodies.

Finally, while TNF-α can signal diverse biological activities, signaling by Fas leads more specifically to cell death.

The foregoing observations together indicate that, unexpectedly, Fas antigen and TNF-R1 induce tumor cell death through distinct signaling pathways.

In one aspect, the invention relates to an in vitro method for cell lysis or inhibition of cell growth by treating cells expressing both the Fas antigen and the type 1 TNF receptor (TNF-R1) with an effective amount of a TNF-R1 agonist and a Fas agonist. The TNF-R1 agonist preferably is TNF-α or an anti-TNF-R1 antibody mimicking the cytotoxic activity of TNF-α. The Fas agonist preferably is an antibody exhibiting cell killing activity.

In another aspect, the invention concerns a bispecific molecule comprising a first contiguous amino acid sequence specifically binding TNF-R1 and a second contiguous amino acid sequence specifically binding Fas antigen. In a preferred embodiment, the bispecific molecule comprises an immunoglobulin sequence, such as an immunoglobulin heavy chain constant domain sequence comprising at least a hinge region and the C_{H}2 and C_{H}3 domains of an IgG-1, IgG-2 or IgG-3 immunoglobulin. In addition to their therapeutic utility, the bispecific molecules of the present invention are also useful for identifying and labeling cell types, in particular malignancies, expressing both TNF-R1 and Fas antigen.

In a further aspect, the invention concerns a nucleotide sequence encoding a bispecific molecule as hereinabove defined.

In a still further aspect, the invention concerns a replicable expression vector containing and capable of expressing, in a suitable host cell, such nucleotide sequence.

In yet another aspect, the invention relates to a host cell transformed with such expression vector, and to the culturing of the transformed host cell so as to express the encoded bispecific molecule.

The invention also concerns a pharmaceutical composition comprising the foregoing bispecific molecule in an amount capable of inducing cell lysis or inhibiting cell growth, in admixture with a pharmaceutically acceptable carrier.

In a further aspect, the invention concerns an in vitro method for cell lysis or inhibition of cell growth by treating cells carrying both the Fas antigen and the type 1 TNF receptor (TNF-R1) with an effective amount of a bispecific molecule as hereinabove defined.

### BRIEF DESCRIPTION OF THE DRAWINGS

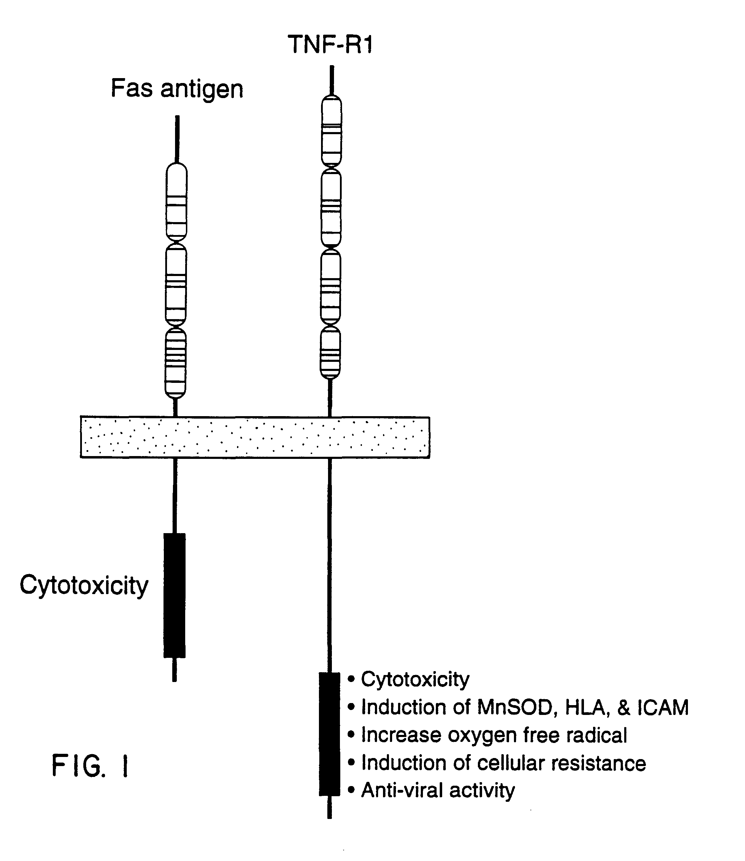

Figure 1 is a schematic diagram of the structure of the Fas antigen and TNF-R1, respectively. It also shows the distinct downstream signaling pathways mediated by Fas antigen and TNF-R1. While TNF-R1 has been shown to mediate cytotoxicity, fibroblast proliferation, induction of MnSOD, HLA and ICAM expression, increase of oxygen free radicals, induction of cellular resistance, and antiviral activity, the biological activity of Fas antigen appears to be limited to the mediation of cytotoxicity.

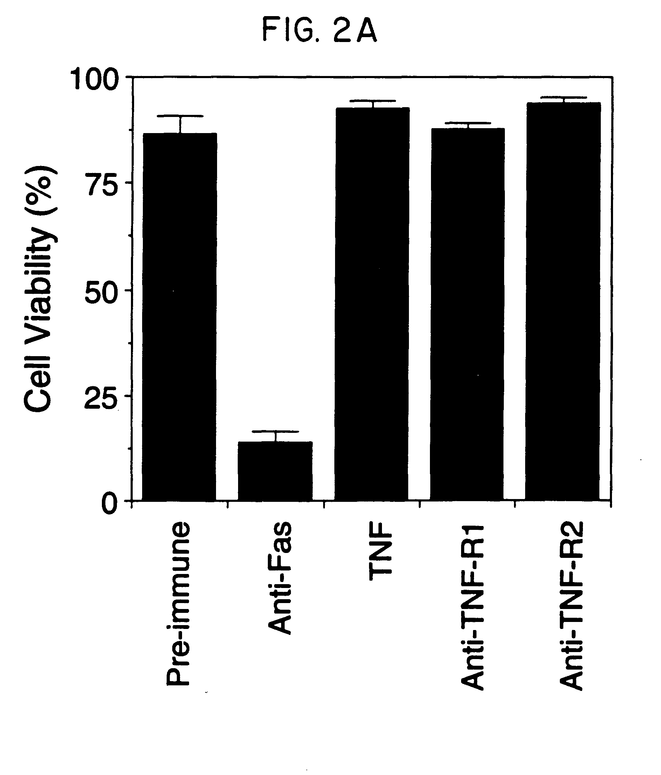

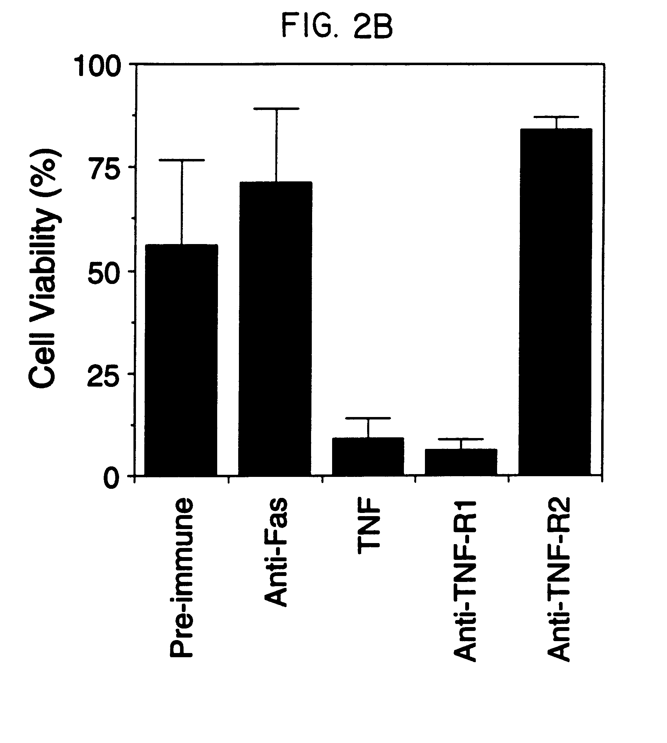

Figures 2a and 2b show the effect of Fas and TNF-R1 agonists on HUT-78 (a) and ME-180 (b) cells. Cells were treated with either TNF-α (1 µg/ml) or various rabbit sera (1:20 dilution) against Fas, TNF-R1 or TNF-R2 or preimmune serum in the presence of CHI for 24 hrs. Cell viability was then determined as described in the legend to Table 1. The relative % viability was mean +/- S,D from six replicates.

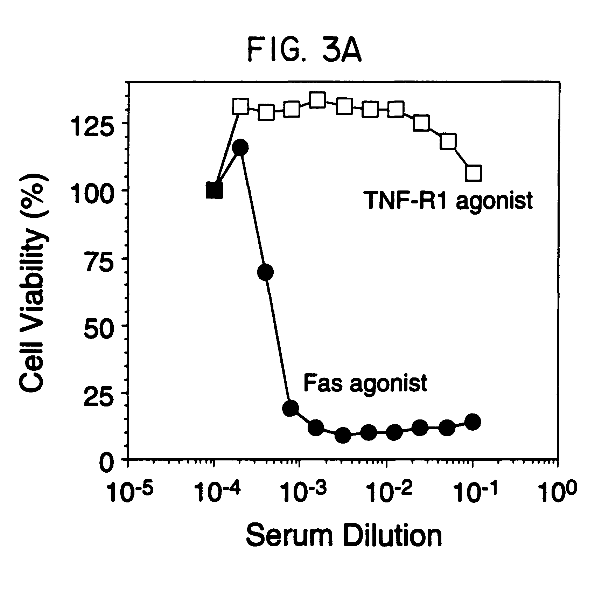

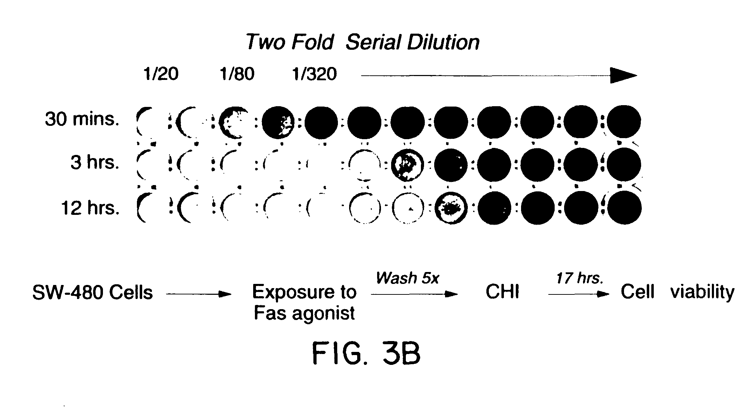

Figures 3a and 3b show that SW-480 cells are killed by Fas but not TNF-R1 agonist. The killing assay (a) was carried out the same way as described in Table 1. Cells were exposed to Fas agonist for different times as indicated (b), washed with PBS five times and further incubated with CHI (1 µg/ml) for 17 hrs before determination of cell viability as described in the legend to Table 1.

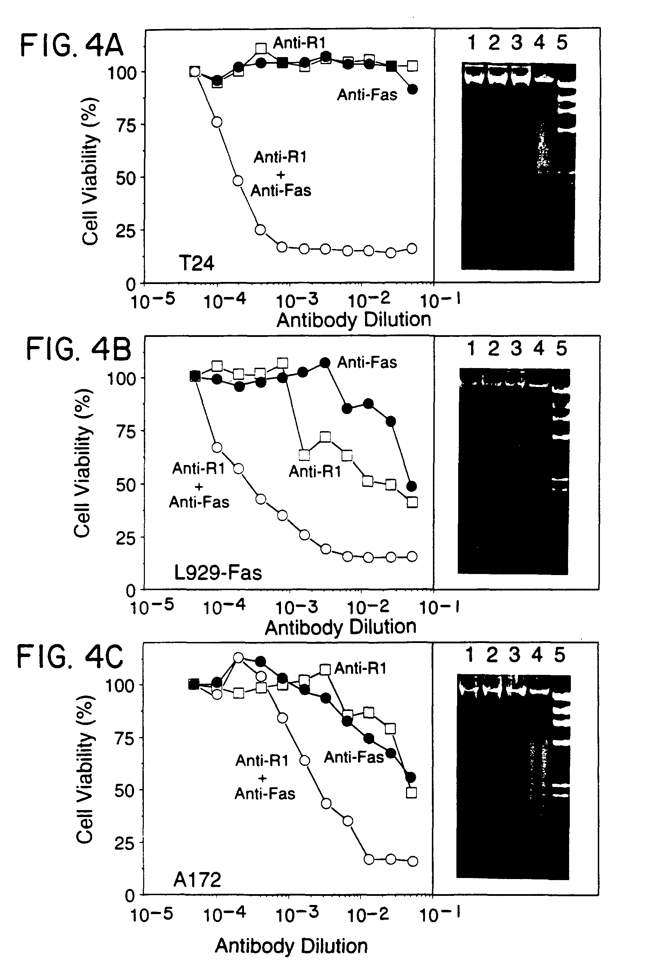

Figure 4 shows synergistic cytotoxicity of agonist antibodies to TNF-R1 and to the Fas antigen, T24 cells (a), murine L929 cells expressing human Fas antigen (b), and A172 cells (c) were treated with either agonist anti-Fas or agonist anti-human TNF-R1 antibodies alone or in combination in the presence of cycloheximide (CHI; 10 µg/ml) and analyzed as described in the Example. Percent viability are mean values obtained from three to six replicates. Anti-murine R1 antibodies were used instead of anti-human TNF-R1 antibodies for the L929 cells. Nuclear DNA was isolated from cells treated with 1/100 dilution of preimmune serum (lane 1); anti-TNFR1 (lane 2); anti-Fas (lane 3) and a combination of anti-TNF-R1 and anti-Fas (lane 4). A Hind III digest of lambda phage DNA was used as molecular weight markers (lane 5).

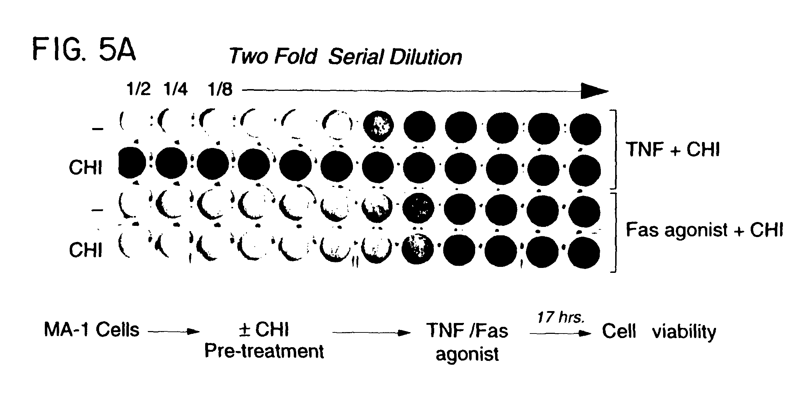

Figure 5(a) shows that pretreatment of MA-1 cells with TNF-R1 but not Fas agonist protects against TNF-α+CHI killing; (b) TNF-α-induced protective proteins do not protect against Fas agonist killing; and (c) CHI pretreatment protects against TNF but not Fas agonist killing. All killing assays were performed as described in the legend to Table 1.

Table 1. Killing various tumor cells by Fas agonist or TNF-R1 agonist.
100 µl of a suspension of cells at 2x10⁵/ml in F12 medium supplemented with 5% FBS was added to each well of a 96-well plate for 24 hours prior to the assay. The test samples (containing various doses of TNF,TNF-R1 agonist or Fas agonist) were added to the first column and the samples were serially 2-fold diluted in medium containing of cycloheximide (CHI) in the absence of FBS. Appropriate amounts of samples in certain concentrations of CHI were added to the attached cells. After 24 hours, wells were checked by microscopic examination to confirm 90-100% lysis. The fluid from all wells was poured off and the attached viable cells were stained with 0.5% crystal violet in 20% methanol for 10-15 minutes at ambient temperature. Cell viability was determined by eluting the dye from the stained cells with 0.1 M sodium citrate /0.1 M citric acid and 50% ethanol and measuring absorbance at 540 nm. The sign (+/-, + , ++, +++) indicates about 25, 50, 75 and 100 % cytotoxicity respectively whereas the sign (-) shows less than 10 % cytotoxicity. The method for staining the level of TNF-R1 or Fas antigen were performed using purified polyclonal rabbit antibodies against either TNF-R1 or Fas antigen was similarly described earlier. All cell lines were originally obtained from the American Type Culture Collection and were free of mycoplasma. All media were supplemented with 5% fetal bovine serum (FBS), 1% L-glutamine, 100 U/ml penicillin and 100 µg/ml streptomycin (Gibco). Cells were incubated at 37° C in a 5% CO₂ atmosphere. Recombinant human TNF-α (4x10⁷ U/mg), was supplied by the Genentech manufacturing group. The rabbit antisera against human TNF-R1 were the same as those described previously [Tartaglia, L.A. and Goeddell, D.V., J. Biol. Chem. 267: 4304-4307 (1992)] and rabbit antisera against soluble IgG-human Fas antigen and were purified by protein A column and were gifts from G. Bennett (Genentech).

### DETAILED DESCRIPTION OF THE INVENTION

### I. Definitions

The term "TNF-R1" refers to a family of polypeptide molecules that comprise the full-length, native amino acid sequence of any TNF-Rls from any species, including the human type 1 TNF receptor having the amino acid sequence disclosed in EP 417,563 (published 20 March 1991), and naturally occurring alleles and variants of such TNF-R1 receptors. This definition specifically encompasses the soluble forms of TNF-R1 receptors, from natural sources, synthetically produced in vitro or obtained by genetic manipulation including methods of recombinant DNA technology, as well as variously glycosylated and unglycosylated forms of native TNF-Rls. TNF-R1 receptors from non-human mammalian (murine, bovine, equine, porcine, etc.) species can, for example, be obtained by cross-species hybridization, using probes obtained from the human DNA sequence as hybridization probes to isolate TNF-R cDNAs from the respective non-human mammalian cDNA libraries.

The terms "Fas receptor" "Fas antigen" "Fas" and "APO-1" are used interchangeably and refer to a family of polypeptide molecules that comprise the full-length, native amino acid sequence of any Fas antigen from any species, including the human Fas antigen having the amino acid sequence disclosed by Itoh *et al.,* Cell 66, 233-243 (1991), and the mouse Fas antigen amino acid sequence reported by Watanabe-Fukunaga *et al*., J. Immunol. 148, 1274-1279 (1992), and naturally occurring alleles and variants of native Fas antigens. This definition specifically encompasses native sequence Fas receptors from natural sources, synthetically produced in vitro or obtained by genetic manipulation including methods of recombinant DNA technology, and variously glycosylated and unglycosylated forms of native Fas receptors. Fas antigen from non-human mammalian (murine, bovine, equine, porcine, etc.) species can, for example, be obtained by cross-species hybridization, using probes obtained from the human DNA sequence as hybridization probes to isolate Fas cDNAs from the respective non-human mammalian cDNA libraries.

The term "agonist" as defined for the purpose of the present invention covers antibodies and other molecules specifically binding to TNF-R1 and Fas receptor, respectively, and, when in combination, capable of inducing death or inhibiting the growth of target cells. The term specifically covers naturally occurring and variant ligands of TNF-R1, such as TNF-α, TNF-β (collectively referred to as TNF) and agonist anti-TNF-R1 antibodies mimicking the cytotoxic activity of TNF, as well as naturally occurring and variant ligands of Fas receptor, known in the art or identified hereinafter, and agonist anti-Fas antibodies capable of lysing or inhibiting the growth of target cells.

"Synergism", "synergistic" and grammatical variants of these phrases in the context of the present invention are defined according to the accepted definition [Goodman *et al.,* The Pharmacological Basis of Therapeutics, McMillan Publishing Co. Inc., New York (1980)]. This is most easily seen in terms of the construction of an "isobologram" which plots the dosage levels required for a specific identical biological response of each of two ingredients along the X and Y axes. While simply additive effects would generate a straight line as one ingredient diminishes and the other increases, synergistic effects can be recognized by the generation of a concave curve, such that only a small increase in one component compensates for a drastic increase in the amount of the other.

The terms "tumor necrosis factor" and "TNF" when used without any affixed Greek character, collectively designate both TNF-α and TNF-β (lymphotoxin) and refer to a family of polypeptide molecules that comprise the full-length, native amino acid sequence of any TNF-α or TNF-β from any species, including human TNF-α having the amino acid sequence disclosed by Pennica *et al.,* Nature 312, 721 (1984), and human TNF-β, also referred to as lymphotoxin, as described by Gray *et al..* Nature 312, 724 (1984), and amino acid sequence variants of such TNF-α and TNF-β molecules, provided that such variants are capable of hybridizing under stringent conditions with the corresponding native amino acid sequence. This definition specifically encompasses TNF-α and TNF-β from natural sources, synthetically produced in vitro or obtained by genetic manipulation including methods of recombinant DNA technology. The amino acid sequence variants preferably share at least about 65% sequence homology, more preferably at least about 75% sequence homology with any domain of a native TNF-α or TNF-β amino acid sequence. The definition specifically covers variously glycosylated and unglycosylated forms of native and variant TNF-α and TNF-β. TNF molecules from non-human mammalian (murine, bovine, equine, porcine, etc.) species can, for example, be obtained by cross-species hybridization, using probes obtained from the human DNA sequence as hybridization probes to isolate Fas cDNAs from the respective non-human mammalian cDNA libraries. TNFs may have more than one receptor binding site. For example, human TNF-α (hTNF-α) has been described to have three receptor-interaction sites [VanOstade *et al.,* The EMBO J. 10, 827 (1991)]. In the context of the present invention binding of all receptor binding sites to TNFR-1 is not required, although interaction with more than one receptor binding site is preferred to maximize binding and biological activity.

The "stringent conditions" are overnight incubation at 42 °C in a solution comprising: 50% formamide, 5xSSC (150 mM NaCl, 15 mM trisodium citrate), 50 mM sodium phosphate (pH 7.6), 5x Denhardt's solution, 10% dextran sulfate, and 20 µg/ml denatured, sheared salmon sperm DNA, followed by washing the filters in 0.1 x SSC at about 65 °C.

The terms "amino acid" and "amino acids" refer to all naturally occurring L-α-amino acids. The amino acids are identified by either the single-letter or three-letter designations:

| | | | | | |
|---|---|---|---|---|---|
| Asp | D | aspartic acid | Ile | I | isoleucine |
| Thr | T | threonine | Leu | L | leucine |
| Ser | S | serine | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |
| Val | V | valine | Gin | Q | glutamine |
| Met | M | methionine | Asn | N | asparagine |

These amino acids may be classified according to the chemical composition and properties of their side chains. They are broadly classified into two groups, charged and uncharged. Each of these groups is divided into subgroups to classify the amino acids more accurately:

### I. Charged Amino Acids

Acidic Residues: aspartic acid, glutamic acid
Basic Residues: lysine, arginine, histidine

### II. Uncharged Amino Acids

Hydrophilic Residues: serine, threonine, asparagine, glutamine
Aliphatic Residues: glycine, alanine, valine, leucine, isoleucine
Non-polar Residues: cysteine, methionine, proline
Aromatic Residues: phenylalanine, tyrosine, tryptophan

The term "amino acid sequence variant" refers to molecules with some differences in their amino acid sequences as compared to a native amino acid sequence.

"Homology" is defined as the percentage of residues in the candidate amino acid sequence that are identical with the residues in the amino acid sequence of their native counterparts after aligning the sequences and introducing gaps, if necessary, to achieve the maximum percent homology. Methods and computer programs for the alignment are well known in the art.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Insertional variants are those with one or more amino acids inserted immediately adjacent to an amino acid at a particular position in a native sequence. Immediately adjacent to an amino acid means connected to either the α-carboxy or α-amino functional group of the amino acid.

Deletional variants are those with one or more amino acids in the native amino acid sequence removed. Ordinarily, deletional variants will have one or two amino acids deleted in a particular region of the molecule.

The term "glycosylation variant" is used to refer to a glycoprotein having a glycosylation profile different from that of a native counterpart. Glycosylation of polypeptides is typically either N-linked or O-linked. N-linked refers to the attachment of the carbohydrate moiety to the side-chain of an asparagine residue. The tripeptide sequences, asparagine-X-serine and asparagine-X-threonine, wherein X is any amino acid except proline, are recognition sequences for enzymatic attachment of the carbohydrate moiety to the asparagine side chain. O-linked glycosylation refers to the attachment of one of the sugars N-acetylgalactosamine, galactose, or xylose to a hydroxyamino acid, most commonly serine or threonine, although 5-hydroxyproline or 5-hydroxylysine may also be involved in O-linked glycosylation. Any difference in the location and/or nature of the carbohydrate moieties present in a TNF-R1 or Fas agonist protein as compared to its native counterpart is within the scope herein.

Antibodies (Abs) and immunoglobulins (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

Native antibodies and immunoglobulins are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one and (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains [Clothia *et al*., J. Mol. Biol. 186, 651-663 (1985); Novotny and Haber, Proc. Natl. Acad. Sci. USA 82, 4592-4596 (1985)].

The variability is not evenly distributed through the variable regions of antibodies. It is concentrated in three segments called complementarity determining regions (CDRs) or hypervariable regions both in the light chain and the heavy chain variable regions. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies [see Kabat, E.A. *et al.*, Sequences of Proteins of Immunological Interest National Institute of Health, Bethesda, MD (1987)]. The constant domains are not involved directly in binding an antibody to an antigen, but exhibit various effector functions, such as participation of the antibody in antibody-dependent cellular toxicity.

Papain digestion of antibodies produces two identical antigen binding fragments, called Fab fragments, each with a single antigen binding site, and a residual "Fc" fragment, whose name reflects its ability to crystallize readily. Pepsin treatment yields an F(ab')₂ fragment that has two antigen combining sites and is still capable of cross-linking antigen.

"Fv" is the minimum antibody fragment which contains a complete antigen recognition and binding site. This region consists of a dimer of one heavy and one light chain variable domain in tight, non-covalent association. It is in this configuration that the three CDRs of each variable domain interact to define an antigen binding site on the surface of the V_{H}-V_{L} dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (C_{H}1) of the heavy chain. Fab' fragments differ from Fab fragments by the addition of a few residues at the carboxy terminus of the heavy chain C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other, chemical couplings of antibody fragments are also known.

The light chains of antibodies (immunoglobulins) from any vertebrate species can be assigned to one of two clearly distinct types, called kappa and lambda (λ), based on the amino acid sequences of their constant domains.

Depending on the amino acid sequence of the constant region of their heavy chains, immunoglobulins can be assigned to different classes. There are five major classes of immunoglobulins: IgA, IgD, IgE, IgG and IgM, and several of these may be further divided into subclasses (isotypes), e.g. IgG-1, IgG-2, IgG-3, and IgG-4; IgA-1 and IgA-2. The heavy chain constant regions that correspond to the different classes of immunoglobulins are called α, delta, epsilon, γ, and µ, respectively. The subunit structures and three-dimensional configurations of different classes of immunoglobulins are well known. IgA-1 and IgA-2 are monomeric subclasses of IgA, which usually is in the form of dimers or larger polymers. Immunocytes in the gut produce mainly polymeric IgA (also referred to poly-IgA including dimers and higher polymers). Such poly-IgA contains a disulfide-linked polypeptide called the "joining" or "J" chain, and can be transported through the glandular epithelium together with the J-chain-containing polymeric IgM (poly-IgM), comprising five subunits.

The term "antibody" is used in the broadest sense and specifically covers single anti-TNF-R1 and anti-Fas agonist monoclonal antibodies and anti-TNF-R1 and anti-Fas agonist antibody compositions with polyepitopic specificity.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins.

The monoclonal antibodies herein include hybrid and recombinant antibodies produced by splicing a variable (including hypervariable) domain of an anti-TNF-R1 or anti-Fas antibody with a constant domain (e.g. "humanized" antibodies), only one of which is directed against TNF-R1 or Fas, or a light chain with a heavy chain, or a chain from one species with a chain from another species, or fusions with heterologous proteins, regardless of species of origin or immunoglobulin class or subclass designation, as well as antibody fragments (e.g., Fab, F(ab')₂, and Fv), so long as they exhibit the desired biological activity. [See, e.g. Cabilly, et al., U.S. Pat. No. 4,816,567; Mage & Lamoyi, in Monoclonal Antibody Production Techniques and Applications, pp.79-97 (Marcel Dekker, Inc., New York, 1987).]

Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler & Milstein, Nature 256:495 (1975), or may be made by recombinant DNA methods [Cabilly, et al., U.S. Pat. No. 4,816,567].

The antibodies herein are preferably against epitopes within the extracellular domains of TNF-R1 and Fas antigen.

The antibodies included within the scope of the invention belong to a subclass or isotype that, upon complexing with a TNF-R1 receptor and a Fas receptor, respectively, induce the killing or growth inhibition of target cells. Monoclonal anti-TNF-R1 agonist antibodies suitable for practicing the present invention include the twelve affinity purified monoclonal antibodies to human TNF-R1 described by Wong *et al*., J. Immunol. 149, 3350-3353 (1992), designated 981, 982, 983, 984, 985, 986, 993, 994, 1002, 1011, 1015, and 1021. These antibodies were produced by hyperimmunizing BALB/c mice in the hind footpads with soluble TNF-R1 (sTNF-R1), in RIBI adjuvant (RIBI ImmunoChem Research, Hamilton, MT) and fusing the draining inguinal and popliteal lymph node cells with the mouse myeloma cell line X63-Ag8.653 [Kearney, J.F. *et al.,* J. Immunol. 123, 1548-1550 (1979)]. The antibodies were purified from ascites fluid using protein A-Sepharose (Repligen Corp., Cambridge, MA) and established affinity chromatography methods [Goding, J.W., J. Immunol. Methods 20, 241-253 (1978)]. Cell lines producing anti-TNF-R1 monoclonal antibodies 981 and 982 were deposited with the American Type Culture Collection, 12301 Parklawn Drive, Rockville, MD, USA (ATCC) on 11 February 1993, under ATCC deposit numbers HB 11266 and HB 11267, respectively. The deposits were made under the provisions of the Budapest Treaty on the International Recognition of the Deposit of Microorganisms for the Purpose of Patent Procedure and the Regulations thereunder (Budapest Treaty).

Anti-Fas monoclonal antibodies having cell-killing (cytolytic) activity are also known in the art [see, for example Yonehara *et al*., J. Exp. Med. 169, 1747 (1989) and Kobayashi et al., Proc. Natl. Acad. Sci. USA 87, 9620-9624 (1990); Köhler et *al.,* Ultratruct. Pathol. 14, 513-518 (1990)].

The phrase "bispecific molecule" is used to define a polypeptide with two specificities. The amino acid sequences providing the two specificities ("binding domains") may be directly fused to each other or may be connected with a "linker". The linker may be the residue of a covalent cross-linking agent capable of linking the two binding domains without the impairment of their ability to bind their respective receptors or a linkage the formation of which is induced by such cross-linking agents. A concise review of covalent cross-linking reagents, including a guide to the selection of such reagents and methods for their preparation are provided by Tae, H. Jr. in Meth. Enzymol. 580-609 (1983) and in the references cited therein. The selection of the most appropriate reagent for a specific purpose from the wide variety of cross-linking agents available, is well within the skill of an ordinary artisan. In general, zero-length, homo- or heterobifunctional cross-linking agents are preferred for making the bispecific molecules of the present invention. Zero-length cross linking reagents induce the direct conjugation of two binding domains without the introduction of any extrinsic material. Agents that catalyze the formation of disulfide bonds belong in this category. Another example is reagents that induce the condensation of carboxy and primary amino groups to form an amide bond, such as carbodiimides, ethylchloroformate, Woodward's reagent K1, carbonyldiimidazole, etc. Homobifunctional reagents carry two identical functional groups, whereas heterobifunctional reagents contain two dissimilar functional groups. A vast majority of the heterobifunctional cross-linking agents contains a primary amine-reactive group and a thiol-reactive group. A novel heterobifunctional linker for formyl to thiol coupling was disclosed by Heindel, N.D. *et al*., Bioconjugate Chem. 2, 427-430 (1991)]. In a preferred embodiment, the covalent cross-linking agents are selected from reagents capable of forming disulfide (-S-S-), glycol (-CH[OH]-CH[OH]-), azo (-N=N-), sulfone (-S[=O₂]-), or ester (-C[=O]-O-) bridges. In a different approach, the binding domains are linked via their oligosaccharides. Chemical or enzymatic oxidation of oligosaccharides on polypeptide ligands to aldehydes yields unique functional groups on the molecule, which can react with compounds containing, for example, amines hydrazines, hydrazides, or semicarbazides. Since the glycosylations sites are well defined in polypeptide molecules, selective coupling via oxidized oligosaccharide moieties will yield in a more uniform product than other coupling methods, and is expected to have less adverse effect on the receptor binding properties of the ligands. Carbohydrate-directed heterobifunctional cross-linking agents are, for example, 4-(4-male imiolophyenyl) butyric acid hydrazide. HCl(MPBH) and 3-(2-pyridyldithio) propionyl hydrazide (PDPH) are commercially available from Pierce Chemical Company, Rockford, Illinois. MPBH was also disclosed by Chamow *et al*., J. Biol. Chem. 267, 15916-15922 (1992). It will be understood that the coupling of more than two binding sequences with various linked sequences, e.g., cross-linking reagents is possible, and is within the scope of the present invention.

In a further embodiment, in the bispecific molecules of the present invention the binding domains are connected by polypeptide linker sequences, and accordingly, are presented to their receptor as a single-chain multifunctional polypeptide molecule. The polypeptide linker functions as a "spacer" whose function is to separate the functional binding domains so that they can independently assume their proper tertiary conformation. The polypeptide linker usually comprises between about 5 and about 25 residues, and preferably contains at least about 10, more preferably at least about 15 amino acids, and is composed of amino acid residues which together provide a hydrophilic, relatively unstructured region. Linking amino acid sequences with little or no secondary structure work well. If desired, one or more unique cleavage sites recognizable by a specific cleavage agent (e.g. protease) may be included in the polypeptide linker. The specific amino acids in the spacer can vary, however, cysteines should be avoided. The spacer sequence can be designed to assume a desired structure, such as a helical structure. Suitable polypeptide linkers are, for example, disclosed in WO 88/09344 (published 1 December 1988), as are methods for the production of multifunctional proteins comprising such linkers.

In a further specific embodiment, the binding domains are connected by amphiphatic helices. It is known that recurring copies of the amino acid leucine (Leu) in gene regulatory proteins can serve as teeth that "zip" two protein molecules together to provide a dimer. Leucine zipper was first discovered when a small segment of the protein C/EBP was fit into a hypothetical alpha helix. Surprisingly, the leucines, which make up every seventh amino acid in this protein, lined up in a column. Subsequently, two additional, C/EBP related proteins were identified and shown to have a similar function. One of them, GCN4 is a gene regulatory protein from yeast, the other one, is the product of a proto-oncogene jun. It has been found that zipper regions associate in parallel when they combine, i.e. the leucines on apposed molecules line up side by side. It has also been shown that non-identical proteins may be zippered to provide heterodimers. Such leucine zippers are particularly suitable for preparing bispecific molecules within the scope of the invention. Alternatively, the sequence of the amphipathic helix may be taken from a four-helix bundle design, essentially as described by Pack P. and Pluckthun, A., Biochemistry 31, 1579-1584 (1992). For further details about molecular, e.g. leucine zippers, which can serve as linkers for the purpose of the present invention, see for example: Landschulz, W.H., *et al*. Science 240, 1759-1764 (1988); O'Shea, E.K. *et al.,* Science 243, 538-542 (1989); McKnight, S.L., Scientific American 54-64, April 1991; Schmidt-Dorr. T. *et al.*, Biochemistry 30, 9657-9664 (1991); Blondel, A. and Bedouelle, H. Protein Engineering 4, 457-461 (1991), Pack, P. and Pluckthun, A., supra, and the references cited in these papers.

In a preferred embodiment, the linker comprises an immunoglobulin sequence preferably resulting in a bispecific antibody or immunoadhesin.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e. is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesins may be obtained from any immunoglobulin, such as IgG-1, IgG-2, IgG-3, or IgG-4 subtypes, IgA (including IgA-1 and IgA-2), IgE, IgD or IgM.

As used herein the phrase "bispecific immunoadhesin" designates immunoadhesins having at least two binding specificities, one of which may be an antigen binding site of an antibody. Bispecific immunoadhesins can generally be assembled as hetero-multimers, and particularly as heterodimers,
-trimers or -tetramers, essentially as disclosed in WO 89/02922 (published 6 April 1989) or in EP 314,317 (published 3 May 1989). Whereas the binding domains providing the two desired specificities in the bispecific molecules of the present invention preferably replace the variable domains of immunoglobulins, they can also be inserted between immunoglobulin heavy chain and light chain sequences such that an immunoglobulin comprising a chimeric heavy chain is obtained. In this embodiment, the binding sequences are fused to the 3' end of an immunoglobulin heavy chain in each arm of an immunoglobulin, either between the hinge and the CH2 domain, or between the CH2 and CH3 domains. Similar constructs have been reported by Hoogenboom, H. R. *et al*., Mol. Immunol. 28, 1027-1037 (1991).

The term "bispecific antibody" is used herein to describe antibody molecules which comprise two different antigen binding sites. The bispecific antibodies may be assembled as hetero-multimers, and particularly as hetero-dimers, -trimers or tetramers, as hereinabove described for bispecific immunoadhesins.

Diagrams illustrating the possible structures of mono- and bispecific immunoadhesins are, for example, disclosed in U.S. Patent No. 5,116,964 issued 26 May 1992, as are chains or basic units of varying structure which may be utilized to assemble the monomers and hetero- and homo-multimers of immunoglobulins in such constructs.

Bispecific immunoadhesins and antibodies are also disclosed in EP 355,068 published 21 February 1990, and PCT application publication no. WO 91/05871 published 2 May 1991.

In certain cases it may be advantageous to prepare trimeric bispecific immunoadhesins, where in one arm of a Y-shaped immunoglobulin, the first heavy chain constant domain (CH1) is removed or altered to eliminate its ability to covalently bind to an immunoglobulin light chain (i.e. the light chain binding site is eliminated or inactivated), while in the other arm the light chain binding site within the CH1 domain is retained, and is covalently linked to an immunoglobulin light chain. In each arm, the heavy chain constant domain sequences are fused to "binding domains" (replacing the heavy chain variable domains) which are different from one another. One of the binding domains is from a TNF-R1 agonist whereas the other binding domain is a Fas agonist as hereinabove defined. The resultant structure is a trimer with two different binding specificities (heterotrimer) composed of an immunoglobulin heavy chain constant domain sequence fused to a first binding domain and a second immunoglobulin heavy chain constant domain sequence fused to a different binding domain and covalently linked to an immunoglobulin light chain. Such trimeric bispecific structures provide preparation and purification advantages over the heterotrimeric structures.

Two or more of the heterotrimers may be covalently linked to each other to provide a multimeric structure. Generally, these assembled bispecific immunoadhesins will have known unit structures. A three-chain unit may be repeated similarly to the higher molecular weight immunoglobulins.

The expression "immunoglobulin heavy chain constant domain sequence lacking a(n immunoglobulin) light chain binding site" is used to designate an immunoglobulin heavy chain constant domain sequence from which sequence elements to which the light chain is ordinarily linked are removed or sufficiently altered (mutated) so that such binding is no longer possible. In a preferred embodiment, the entire CHl domain is deleted but shorter truncations of immunoglobulin constant domains are also suitable, provided that the section to which the light chain is ordinarily disulfide-bonded or interacts with non-covalently is removed. Alternatively, the light chain binding region of an immunoglobulin heavy chain constant domain may be mutated (by substitution or insertion) so that it is no longer capable of covalent or non-covalent binding to an immunoglobulin light chain.

Linear fusion proteins with dual specificity, comprising the fusion of a TNF-R1 agonist or a Fas receptor agonist amino acid sequence to an immunoglobulin sequence may be prepared essentially as described by Traunecker *et al.,* EMBO 10, 3655-3659 (1991).

The terms "nucleic acid molecule encoding", "DNA sequence encoding", and "DNA encoding" refer to the order or sequence of deoxyribonucleotides along a strand of deoxyribonucleic acid. The order of these deoxyribonucleotides determines the order of amino acids along the polypeptide chain. The DNA sequence thus codes for the amino acid sequence.

Nucleic acid is "operably linked" when it is placed into a functional relationship with another nucleic acid sequence. For example, DNA for a presequence or secretory leader is operably linked to a DNA encoding a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, then synthetic oligonucleotide adaptors or linkers are used in accord with conventional practice.

The terms "replicable expression vector" and "expression vector" refer to a piece of DNA, usually double-stranded, which may have inserted into it a piece of foreign DNA. Foreign DNA is defined as heterologous DNA, which is DNA not naturally found in the host cell. The vector is used to transport the foreign or heterologous DNA into a suitable host cell. Once in the host cell, the vector can replicate independently of the host chromosomal DNA, and several copies of the vector and its inserted (foreign) DNA may be generated. In addition, the vector contains the necessary elements that permit translating the foreign DNA into a polypeptide. Many molecules of the polypeptide encoded by the foreign DNA can thus be rapidly synthesized.

In the context of the present invention the expressions "cell", "cell line", and "cell culture" are used interchangeably, and all such designations include progeny. It is also understood that all progeny may not be precisely identical in DNA content, due to deliberate or inadvertent mutations. Mutant progeny that have the same function or biological property as screened for in the originally transformed cell are included.

### 2. Availability of TNF-R1 agonists

TNF-α and TNF-β, the naturally occurring ligands of TNF-R1, are known in the art and are commercially available. TNF-α is, for example, described in EP 168,214 together with methods for its synthesis in recombinant host cell culture. TNF-β (previously called lymphotoxin) and suitable recombinant synthesis methods are described in EP 164,965. The TNF-α and TNF-β described in these applications include cytotoxic amino acid sequence and glycosylation variants which also are used herein.

Poly- and monoclonal antibodies specifically binding TNF-R1 and mimicking the cytotoxic activity of TNF have been described [see e.g. Engelman *et al*., J. Biol. Chem. 265, 14497-14504 (1990)] and are available from the ATCC or other culture collections.

Antibodies specifically binding the Fas antigen and stimulating apoptotic cell death in susceptible tumor cells are known in the art [see, e.g. Yonehara *et al.,* J. Exp. Med. 169, 1747-1756 (1989)].

The following is a brief discussion of certain commonly used techniques that can be used for making such antibodies. Further details of these and similar techniques are found in general textbooks, such as, for example, Cabilly, *et al.,* U. S. Patent No. 4,816,567; Mage & Lamoyi, supra; Sambrook *et al.,* Molecular Cloning: A laboratory Manual, Second Edition, Cold Spring Harbor Laboratory Press, New York, 1989; and Current Protocols in Molecular Biology, Ausubel *et al.* eds., Green Publishing Associates and Wiley-Interscience, 1991.

Anti-TNF-R1 and anti-Fas receptor antibodies acting as agonists of cell killing or cell growth inhibition mediated by these receptors may be produced by any method known in the art. For example, the monoclonal antibodies to be used in accordance with the present invention may be made by the hybridoma method first described by Kohler & Milstein, Nature 256:495 (1975), or may be made by recombinant DNA methods [Cabilly, et al., supra].

In the hybridoma method, a mouse or other appropriate host animal, such as hamster is immunized with a human TNF-R1 or Fas receptor protein by subcutaneous, intraperitoneal, or intramuscular routes to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell [Goding, J. Monoclonal Antibodies : Principles and Practice, pp.59-103 (Academic Press, 1986)].

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred myeloma cells are those that fuse efficiently, support stable high level expression of antibody by the selected antibody-producing cells, and are sensitive to a medium such as HAT medium. Among these, preferred myeloma cell lines are murine myeloma lines, such as those derived from MOPC-21 and MPC-11 mouse tumors available from the Salk Institute Cell Distribution Center, San Diego, California USA, and SP-2 cells available from the American Type Culture Collection, Rockville, Maryland USA. Human myeloma and mouse-human heteromyeloma cell lines also have been described for the production of human monoclonal antibodies. Kozbor, J. Immunol. 133:3001 (1984). Brodeur, et al., Monoclonal Antibody Production Techniques and Applications, pp.51-63 (Marcel Dekker, Inc., New York, 1987).

Culture medium in which hybridoma cells are growing is assayed for production of monoclonal antibodies directed against TNF-R1 or Fas receptor. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA). The monoclonal antibodies for use in the method and compositions of the invention are those that preferentially immunoprecipitate TNF-R1 or Fas receptor that is present in a test sample, or that preferentially bind to TNF-R1 or Fas receptor in a binding assay, and are exhibit cytotoxic activity in susceptable target cells.

After hybridoma cells are identified that produce antibodies of the desired specificity, affinity, and activity, the clones may be subcloned by limiting dilution procedures and grown by standard methods (Goding, J., Supra). Suitable culture media for this purpose include, for example, Dulbecco's Modified Eagle's Medium or RPMI-1640 medium. In addition, the hybridoma cells may be grown in vivo as ascites tumors in an animal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography. Methods for purification of monoclonal antibodies are well known in the art, and are, for example disclosed in Unit 11.11 of "Current Protocols in Molecular Biology", supra, and in the references cited therein.

The amount of a specific antibody present in a hybridoma supernatant can be quantitated by either solid-phase radioimmunoassay (RIA) or by direct enzyme-linked immunoabsorbent assay (ELISA). In the solid-phase radioimmunoassay, serially diluted antiserum is incubated in microtiter wells previously coated with IFN-γ or IFN-γ receptor. Bound antibody is detected by employing ¹²⁵I-labeled anti-immunoglobulin antibodies. The amount of the specific antibody in the antiserum is then determined from a standard curve generated with a specific antibody of known concentration. The unknown antiserum and the standard antibody are assayed in parallel. Protocols for the RIA procedure as used for isotype determination, and the ELISA procedure are, for example, available from Section V of "Current Protocols in Molecular Biology", supra, and from the references cited therein.

DNA encoding the monoclonal antibodies useful in the method of the invention is readily isolated and sequenced using conventional procedures (e.g., by using oligonucleotide probes that are capable of binding specifically to genes encoding the heavy and light chains of murine antibodies). The hybridoma cells described hereinabove serve as a preferred source of such DNA. Once isolated, the DNA may be placed into expression vectors, which are then transfected into host cells such as simian COS cells, Chinese Hamster ovary (CHO) cells, or myeloma cells that do not otherwise produce immunoglobulin protein, to obtain the synthesis of monoclonal antibodies in the recombinant host cells.

Amino acid sequence, glycosylation variants and covalent derivatives of any native or recombinant TNF-R1 agonist species, such as TNF can be prepared by methods known in the art. Generally, particular regions or sites of the DNA encoding TNF will be targeted for mutagenesis, and thus the general methodology employed to accomplish this is termed site-directed mutagenesis. The mutations are made using DNA modifying enzymes such as restriction endonucleases (which cleave DNA at particular locations), nucleases (which degrade DNA) and/or polymerases (which synthesize DNA). Restriction endonuclease digestion of DNA followed by ligation may be used to generate deletions, as described in section 15.3 of Sambrook et al., Supra.

Oligonucleotide-directed mutagenesis is the preferred method for preparing the substitution variants of TNF. It may also be used to conveniently prepare the deletion and insertion variants that can be used in accordance with this invention. This technique is well known in the art as described by Adelman et al. (DNA, 2:183 [1983]). The oligonucleotides are readily synthesized using techniques well known in the art such as that described by Crea et al. (Proc. Nat'l. Acad. Sci. USA, 75:5765 [1978]). The production of single-stranded templates for use in this technique is described in sections 4.21-4.41 of Sambrook et al., Supra.

PCR mutagenesis is also suitable for making the TNF variants that can be used in the methods of the present invention. The PCR technique is, for example, disclosed in U.S. Patent No. 4,683,195, issued 28 July 1987, in section 14 of Sambrook *et al.,* Molecular Cloning: A Laboratory Manual, second edition, Cold Spring Harbor Laboratory Press. New York; 1989, or in Chapter 15 of Current Protocols in Molecular Biology, Ausubel *et al.* eds., Greene Publishing Associates and Wiley-Interscience 1991.

The DNA encoding the TNF variants hereof is inserted into a replicable expression vector for further cloning and expression. Many vectors are available, and selection of the appropriate vector will depend on 1) whether it is to be used for DNA amplification (cloning) or for expression, 2) the size of the DNA to be inserted into the vector, and 3) the host cell to be transformed with the vector. Each vector contains various components depending on its function and the host cell with which it is compatible. The vector components generally include, but are not limited to, one or more of the following: a signal sequence, an origin of replication, one or more marker genes, an enhancer element, a promoter and a transcription terminator sequence.

Suitable vectors are prepared using standard recombinant DNA procedures. Isolated plasmids and DNA fragments are cleaved, tailored, and ligated together in a specific order to generate the desired vectors.

After ligation, the vector with the foreign gene inserted is transformed into a suitable host cell. The transformed cells are selected by growth on an antibiotic, commonly tetracycline (tet) or ampicillin (amp), to which they are rendered resistant due to the presence of tet and/or amp resistance genes on the vector. If the ligation mixture has been transformed into a eukaryotic host cell, transformed cells may be selected by the DHFR/MTX system described above. The transformed cells are grown in culture and the plasmid DNA (plasmid refers to the vector ligated to the foreign gene of interest) is then isolated. This plasmid DNA is then analyzed by restriction mapping and/or DNA sequencing. DNA sequencing is generally performed by either the method of Messing et al., Nucleic Acids Res., 9:309 (1981) or by the method of Maxam et al., Methods of Enzymology, 65:499 (1980).

Multicellular organisms are preferred as hosts to prepare TNF variants. While both invertebrate and vertebrate cell cultures are acceptable, vertebrate cell cultures, particularly mammalian cultures, are preferable. In addition to multicellular eukaryotes, eukaryotic microbes such as filamentous fungi or yeast are suitable to prepare TNF variants. Saccharomyes cerevisiae, or common baker's yeast, is the most commonly used among lower eukaryotic host microorganisms. Prokaryotes are particularly useful for rapid production of large amounts of DNA, for production of single-stranded DNA templates used for site-directed mutagenesis, for screening many mutants simultaneously, and for DNA sequencing of the mutants generated. Suitable prokaryotic host cells include, but are not limited to, various E. coli strains.

Further details of the techniques and materials suitable for preparing the TNF amino acid sequence variants hereof are, for example, disclosed in U.S. Patent No. 5,108.901 issued 28 April 1992, and in European Publication No. 146,354.

The glycosylation variants of native TNF and its amino acid sequence variants are also prepared by techniques known in the art. Glycosylation of polypeptides is typically either N-linked or O-linked. O-linked glycoslation sites may, for example, be modified by the addition of, or substitution by, one or more serine or threonine residue to the amino acid sequence of a polypeptide. For ease, changes are usually made at the DNA level, essentially using the techniques known for the preparation of amino acid sequence variants.

Chemical or enzymatic coupling of glycosydes to TNF or its amino acid sequence variants may also be used to modify or increase the number or profile of carbohydrate substituents. These procedures are advantageous in that they do not require production of the polypeptide that is capable of O-linked (or N-linked) glycosylation. Depending on the coupling mode used, the sugar(s) may be attached to (a) arginine and histidine, (b) free carboxyl groups, (c) free hydroxyl groups such as those of cysteine, (d) free sulfhydryl groups such as those of serine, threonine, or hydroxyproline, (e) aromatic residues such as those of phenylalanine, tyrosine, or tryptophan or (f) the amide group of glutamine. These methods are described in WO 87/05330 (published 11 September 1987), and in Aplin and Wriston, CRC Crit. Rev. Biochem., pp. 259-306 (1981).

Carbohydrate moieties present on TNF or an amino acid sequence variant thereof may also be removed chemically or enzymatically. Chemical deglycosylation requires exposure to trifluoromethanesulfonic acid or an equivalent compound. This treatment results in the cleavage of most or all sugars, except the linking sugar, while leaving the polypeptide intact. Chemical deglycosylation is described by Hakimuddin *et al.,* Arch. Biochem. Biophys. 259, 52 (1987) and by Edge *et al*., Anal. Biochem. 118, 131 (1981). Carbohydrate moieties can be removed by a variety of endo- and exoglycosidases as described by Thotakura *et al*., Meth. Enzymol. 138, 350 (1987). Glycosylation is suppressed by tunicamycin as described by Duskin *et al.,* J. Biol. Chem. 257, 3105 (1982). Tunicamycin blocks the formation of protein-N-glycosydase linkages.

Glycosylation variants can also be produced by selecting appropriate host cells. Yeast, for example, introduce glycosylation 'which varies significantly from that of mammalian systems. Similarly, mammalian cells having a different species (e.g. hamster, murine, insect, porcine, bovine or ovine) or tissue (e.g. lung, liver, lymphoid, mesenchymal or epidermal) origin than the source of the selectin variant, are routinely screened for the ability to introduce variant glycosylation.

Amino acid sequence and glycosylation variants of agonist anti-TNF-R1 antibodies can be made in an analogous manner.

The use of covalent derivatives of TNF and its amino acid sequence and glycosylation variants is within the scope hereof. Such modifications are introduced by reacting targeted amino acid residues of TNF or the TNF variant with an organic derivatizing agent that is capable of reacting with selected side chains or terminal residues, or by harnessing mechanisms of post-translational modification that function in selected recombinant host cells. Covalent derivatization may, or improve properties like half-life, stability, etc. of the molecule. Such modifications are within the ordinary skill in the art and are performed without undue experimentation. Certain post-translational derivatization are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions. Either form of these residues fall within the scope of the invention. Other post-translational modifications include hydroxylation of proline and lysine, phosphorylation of hydroxyl groups of seryl and threonyl residues, methylation of the a-amino groups of lysine, arginine, and histidine side chains [T.E. Creighton, Proteins: Structure and Molecular Properties, W.H. Freeman Co., San Francisco pp. 79-86 (1983)], acetylation of'the N-terminal amines and, in some instances, amidation of the C-terminal carboxyl of IFN-γ. Other covalent derivatives comprise IFN-y covalently bonded to a nonproteinaceous polymer, such as polyethylene glycol, polypropylene glycol or polyoxyalkylenes, in the manner set forth in U.S. Patent Nos. 4,640,835; 4,496,689; 4,301,144; 4,670,417; 4,791,192, 4,179,337, or 5,116,964.

A specific group of TNF variants includes the fusion of a contiguous TNF amino acid sequence mimicking the cytotoxic action of native TNF to a stable plasma protein, such as an immunoglobulin. The resultant molecules are commonly referred to as TNF-immunoglobulin chimeras or, more recently, immunoadhesins.

Ordinarily, the C-terminus of a contiguous amino acid sequence of a receptor binding domain of TNF is fused to the N-terminus of a contiguous amino acid sequence of an immunoglobulin constant region, in place of the variable region(s), however N-terminal fusions are also possible.

Typically, such fusions retain at least functionally active hinge, CH2 and CH3 domains of the constant region of an immunoglobulin heavy chain. Fusions are also made to the C-terminus of the Fc portion of a constant domain, or immediately N-terminal to the CH1 of the heavy chain or the corresponding region of the light chain. This ordinarily is accomplished by constructing the appropriate DNA sequence and expressing it in recombinant cell culture. Alternatively, such structures may be synthesized according to known methods.

The precise site at which the fusion is made is not critical; particular sites are well known and may be selected in order to optimize the biological activity, secretion or binding characteristics.

In a preferred embodiment, the C-terminus of a contiguous amino acid sequence which comprises the binding site(s) for TNF-R1 is fused, at the N-terminal end, to the C-terminal portion of an antibody (in particular the Fc domain), containing the effector functions of an immunoglobulin, e.g. immunoglobulin G₁ (IgG-1). As hereinabove mentioned, it is possible to fuse the entire heavy chain constant region to the sequence containing the binding site(s). However, more preferably, a sequence beginning in the hinge region just upstream of the papain cleavage site (which defines IgG Fc chemically; residue 216, taking the first residue of heavy chain constant region to be 114 [Kobet *et al.,* supra], or analogous sites of other immunoglobulins) is used in the fusion. The immunoglobulin heavy chain constant domain sequence used in the construction of the TNF-immunoglobulin fusions (immunoadhesins) may be devoid of a light chain binding site. This can be achieved by removing or sufficiently altering immunoglobulin heavy chain sequence elements to which the light chain is ordinarily linked so that such binding is no longer possible. Thus, the CH1 domain can be entirely removed in certain embodiments.

In a particularly preferred embodiment, the amino acid sequence containing one or more TNF-R1 domains of a TNF molecule is fused to the hinge region and CH2, CH3: or CH1, hinge, CH2 and CH3 domains of an IgG-1, IgG-2, IgG-3, or IgG-4 heavy chain.

In some embodiments, the TNF-immunoglobulin fusion molecules (immunoadhesins) are assembled as monomers, dimers or multimers, and particularly as dimers or tetramers. Generally, these assembled immunoadhesins will have known unit structures similar to those of the corresponding immunoglobulins. A basic four chain structural unit (a dimer of two immunoglobulin heavy chain-light chain pairs) is the form in which IgG, IgA and IgE exist. A four chain unit is repeated in the high molecular weight immunoglobulins; IgM generally exists as a pentamer of basic four-chain units held together by disulfide bonds. IgA globulin, and occasionally IgG globulin, may also exist in a multimeric form in serum. In the case of multimers, each four chain unit may be the same or different.

It is not necessary that the entire immunoglobulin portion of the TNF-immunoglobulin chimeras be from the same immunoglobulin. Various portions of different immunoglobulins may be combined, and variants and derivatives of native immunoglobulins can be made as hereinabove described with respect to TNF, in order to optimize the properties of the immunoadhesin molecules. For example, immunoadhesin constructs in which the hinge of IgG-l was replaced with that of IgG-3 were found to be functional and showed pharmacokinetics comparable to those of immunoadhesins comprising the entire IgG-1 heavy chain.

Fusion proteins comprising other stable plasma proteins can be made by methods known in the art. For example, fusion proteins comprising N-terminal fragments of human serum albumin (HSA) as stable plasma protein are disclosed in EP 399,666 published 28 November 1990.

### 3. Bispecific antibodies and immunoadhesins

Diagrams illustrating the possible structures of mono- and bispecific immunoadhesins are, for example, disclosed in U.S. Patent No. 5,116,964 issued 26 May 1992, as are chains or basic units of varying structure which may be utilized to assemble the monomers and hetero- and homo-multimers of immunoglobulins in such constructs.

Bispecific immunoadhesins and antibodies are also disclosed in EP 355,068 published 21 February 1990, and PCT application publication no. WO 91/05871 published 2 May 1991.

In a specific embodiment, fusion proteins with two specificities may be made by fusing a TNF amino acid sequence to the 3' end of an antibody heavy chain, where the antibody is an anti-Fas antibody. The TNF amino acid sequence may, for example, be inserted either between the hinge and CH2 domains, or between the CH2 and CH3 domains of the immunoglobulin heavy chain. The chimeric immunoglobulin heavy chain-TNF gene may then be introduced into an immunoglobulin light chain secreting transfectoma cell line, producing the light chain of the desired antibody. The construction of similar structures was reported by Hoogenboom, H.R., Mol. Immunol. 28, 1027-1037 (1991).

Linear fusion proteins with dual specificity (comprising a TNF-R1 agonist and a Fas agonist amino acid sequence) comprising an immunoglobulin sequence may be prepared essentially as described by Traunecker et al., EMBO 10, 3655-3659 (1991). Traunecker *et al.* designed a single-chain polypeptide, containing the FvCD3, the two N-terminal domains of CD4 and C-kappa, designated Janusin (CD4-FvCD3-Ckappa). This bispecific linear molecule was purified by using anti-kappa affinity columns. Linear bispecific molecules within the scope of the present invention can be made in an analogous manner.

The selection of the immunoglobulin that provides the (heavy chain) constant domain sequences in the bispecific immunoadhesins (or TNF-immunoglobulin molecules) of the present invention, is a matter of choice, largely depending on the motivation for constructing them. If the extension of plasma half-life of the agonist is a consideration, immunoglobulins of IgG-1, IgG-2 and IgG-4 isotypes are good candidates, as they all have in vivo half-lives of 21 days, as opposed to IgG-3 which has an in vivo half-life of 7 days. Further differences that might be advantages or detriments in certain situations are, for example, in complement activation. IgG-1, IgG-2 and IgG-3 all activate complement, however, IgG-2 is significantly weaker at complement activation than IgG-1 and does not bind to Fc receptors on mononuclear cells or neutrophils, while IgG-3 shows better complement activation than IgG-1. IgG-1 has only four serologically-defined allotypic sites, two of which (Glm1 and 2) are in the Fc portion; for two of these sites (Glml and 17) one allotype is non-immunogenic. In contrast, there are 12 serologically defined allotypes in IgG-3, all of which are in the Fc portion, only three of which (G3m5, 11 and 21) have an allotype which is non-immunogenic. Thus, for repeated and long-term therapeutic applications, immunoadhesins with IgG-1 derived constant domain sequences are preferred. It is possible to use immunoglobulins from different classes or isotypes in the two arms of the bispecific immunoadhesin molecule. It is further possible to combine sequences from various immunoglobulin classes or isotypes in the same arm of a bispecific molecule, or in both arms of a mono- or bispecific immunoadhesin.

The bispecific immunoadhesins or antibodies may be prepared by standard techniques of recombinant DNA technology detailed hereinabove. Another alternative is to chemically synthesize the gene encoding the chimeras using one of the methods described in Engels et al., Agnew, Chem. Int. Ed. Engl. 28, 716 (1989). These methods include triester, phosphite, phosphoramidite and H-phosphonate methods, PCR and other autoprimer methods, and oligonucleotide syntheses on solid supports.

### 4. Pharmaceutical compositions

The TNF-R1 agonists, Fas agonists and bispecific molecules of the present invention are usually administered as pharmaceutical compositions, usually formulated in dosage forms by methods known in the art; for example, see Remington's Pharmaceutical Sciences, Mack Publishing Company, Easton, Pennsylvania, 15th Edition 1975. The agonists and bispecific molecules of the invention may be administered to a mammal, preferably a human, in a pharmaceutically acceptable dosage form, including those that may be administered to a human intravenously as a bolus or by continuous infusion over a period of time, by intramuscular, subcutaneous, intra-articular, intrasynovial, intrathecal, oral, topical, or inhalation routes.

Such dosage forms encompass pharmaceutically acceptable carriers that are inherently nontoxic and nontherapeutic. Examples of such carriers include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts, or electrolytes such as protamine sulfate, disodium hydrogen phosphate, potassium hydrogen phosphate, sodium chloride, zinc salts, colloidal silica, magnesium trisilicate, polyvinyl pyrrolidone, cellulose-based substances, and polyethylene glycol. Carriers for topical or gel-based forms include polysaccharides such as sodium carboxymethylcellulose or methylcellulose, polyvinylpyrrolidone, polyacrylates, polyoxyethylene-polyoxypropylene-block polymers, polyethylene glycol, and wood wax alcohols. For all administrations, conventional depot forms are suitably used. Such forms include, for example, microcapsules, nano-capsules, liposomes, plasters, inhalation forms, nose sprays, and sublingual tablets. Antibodies typically be formulated in such vehicles at a concentration of about 0.1 mg/ml to 100 mg/ml, and may be administered to induce apoptosis, for example, as described in PCT patent application publication No. WO 91/10448 published 25 July 1991.

TNF is prepared for administration by mixing with physiologically acceptable carriers, such as those hereinabove described. TNF is placed into sterile, isotonic formulations together with required cofactors. The formulation of TNF is preferably liquid, and is ordinarily a physiologic salt solution or dextrose solution, together with conventional stabilizers and/or excipients. The composition may also be provided a lyophilized powder for ultimate delivery in solution. Saline is a suitable carrier, although other conventional parenteral solutions or buffers are usable.

Treatment with a TNF-R1 agonist and a Fas agonist may be performed simultaneously or in tandem, in optional order. If desired, the TNF-R1 agonist and the Fas agonist may be combined in the same pharmaceutical formulation, it is, however, clear that the effectiveness and the synergism exhibited by such combinations is independent of the manner of mixing and of the temporal mode of treatment within at least a relatively short time frame.

The therapeutically effective dosage of TNF when administered alone to a human patient ranges from about 1 to 250 µg/m² per dose, and preferably from about 1 to 10µg/m², and most preferably about 10 µg/m², although the TNF dose depends on the patient, the properties of the TNF employed, e.g. its activity and biological half-life, the concentration of TNF in the formulation, the rate of dosage, the clinical tolerance of the patients involved, the pathological condition of the patients and the like, as is well within the skill of the physician. It will be appreciated that the practitioner will adjust the therapeutic dose in line with clinical experience for any given TNF. The anti-TNF-R1 ad anti-Fas agonist antibodies herein are believed to be effective in a similar dose range, when administered alone.

In accordance with the present invention, the TNF-R1 agonist and the Fas agonist is administered in combination (including simultaneous and tandem administration in optional order). If the TNF-R1 agonist and the Fas agonist exhibit synergism in killing a specific target cell, the effective dose of each component in the combination will be lower than alone. The TNF-R1 agonist and the Fas agonist are typically administered in a percentage proportion of about 0.1 : 100 - 100 : 0.1, preferably 0.1 : 10 - 10 : 0.1, most preferably about 1 : 1 to achieve synergism. These proportions, of course, are subject to modification as required by therapeutic experience.

Cell types particularly susceptible to killing by TNF-R1 agonists and Fas agonists are known in the art, and include lymphoid cell malignancies which express both TNF-R1 and Fas antigen, e.g. various malignancies of B or T cell lymphocytes, including adult T cell leukaemia, as well as lung fibroblast cells, cervical carcinoma cells, breast carcinoma cells, and other malignancies, provided that they express both TNF-R1 and Fas.

Further details of the invention will be apparent from the following, non-limiting example.

### EXAMPLE

### Materials and Methods

### Cells and reagents.

Cell lines were obtained from the American Type Culture Collection, and were free of mycoplasma. The rabbit anti-TNF-R1 antisera against human and murine TNF-R1 have been described previously [see Tartaglia and Goeddel, J. Biol. Chem. 267, 4304 (1992) and Tartaglia *et al.,* Proc. Natl. Acad. Sci. USA 88, 9292 (1991)]. The agonist antibodies were generated against the soluble extracellular domains of the respective receptors. The titers of the antisera were quantitated by a direct antigen-coated enzyme linked immunosorbent assay (ELISA). The dilution of agonist anti-TNF-R1 antibodies giving 50% binding to the purified soluble receptor was 1:109,000. A recombinant Fas-IgG fusion protein that contained the extracellular domain of the Fas antigen was used to generate the rabbit anti-Fas antiserum. The titre of the anti-Fas antiserum was 1:80,000 as quantitated by a direct ELISA.

*The detection of surface receptor expression.*

The presence of cell surface TNF-R1 and Fas antigen was determined by immunofluorescence using the anti-Fas and anti-TNF-Rl antisera and PE-conjugated goat anti-rabbit immunoglobulin (Caltag) and analyzed in an Epics Elite flow cytometer.

### Cytotoxic assay.

A 100 µl suspension of 2 x 10⁴ cells in either F12 or RPMI-1640 medium supplemented with 5% fetal bovine serum (FBS), was added to each well of 96-well plates 24 hours prior to the assay. The test samples containing various doses of anti-Fas and anti-TNF-R1 antibodies and 10 µg/ml cycloheximide (CHI) were added to the target cells. After 17-24 hours at 38.4 °C, cells were stained with 0.5% crystal violet in 20% methanol. Cell viability was determined by eluting the dye from the stained cells with 0.1 M sodium citrate/0.1 M citric acid and 50% ethanol and absorbance was measured at 540 nm. In the case of non-adherent cell lines such as HuT-78, Jurkat and Su-4, cell viability was determined using the Cell Titre 96 kit (Promega).

### Isolation of nuclear DNA.

10⁸ cells were lysed at 4°C in 500 µl of 10 mM Tris-HCl (pH 7.5), 0.1 M NaCl, 5mM MgCl₂ and 0.5% NP-40 for one minutes. Lusates were microfuged at 14 K for one minute in order to pellet the nuclei. Nuclei were treated with 200 mM Tris-HCl (pH 8.5), 100 mM EDTA, 200 µg/ml proteinase K and 1% SDS at 65°C for 2 hours. DNA samples were extracted with phenol/chloroform (1:1). Following treatment with RNase A (100 µg/ml) for one hour at 37°C, DNA was extracted with phenol:chloroform and ethanol precipitated. Approximately 10 µg samples of DNA were analyzed by 1% agarose gel electrophoresis, stained with ethidium bromide and photographed under ultraviolet light.

### Results and Discussion

It has been previously shown that Fas antigen can signal a programmed cell death very similar to that signaled by TNF-R1. To determine whether Fas antigen and TNF-R1 signal cell killing through the same or distinct signaling pathways, we screened a large number of both TNF resistant and TNF sensitive cell lines for killing by agonist anti-Fas polyclonal antibodies (hereinafter referred to as Fas agonists).

In this screen, both SW480 and Hut-78 cells were identified as highly sensitive to Fas agonists. anti-Fas agonist antibodies significantly killed HUT-78 cells whereas pre-immune serum had no effect (Fig. 2a). This activity in the serum can be neutralized by soluble IgG-HuFas immunoadhesin. Interestingly, HUT-78 cells are not killed by TNF-α or anti-TNF-Rl agonist antibodies. This inability of TNF-α to kill HUT-78 cells was not due to the absence of functional TNF-R1, because TNF-α can efficiently induce MnSOD in HUT-78 cells. Therefore, in cells which co-express both functional Fas and TNF-a receptors, only Fas receptor can signal cytotoxicity. Similar results were also observed in SW-480 cells (Fig. 3a and 3b).

We also identified several cell lines that were highly sensitive to TNF-a and anti-TNF-R1 agonist antibodies, but showed no sensitivity to agonist antibodies specifically binding Fas (Fig. 2b and Table 1).

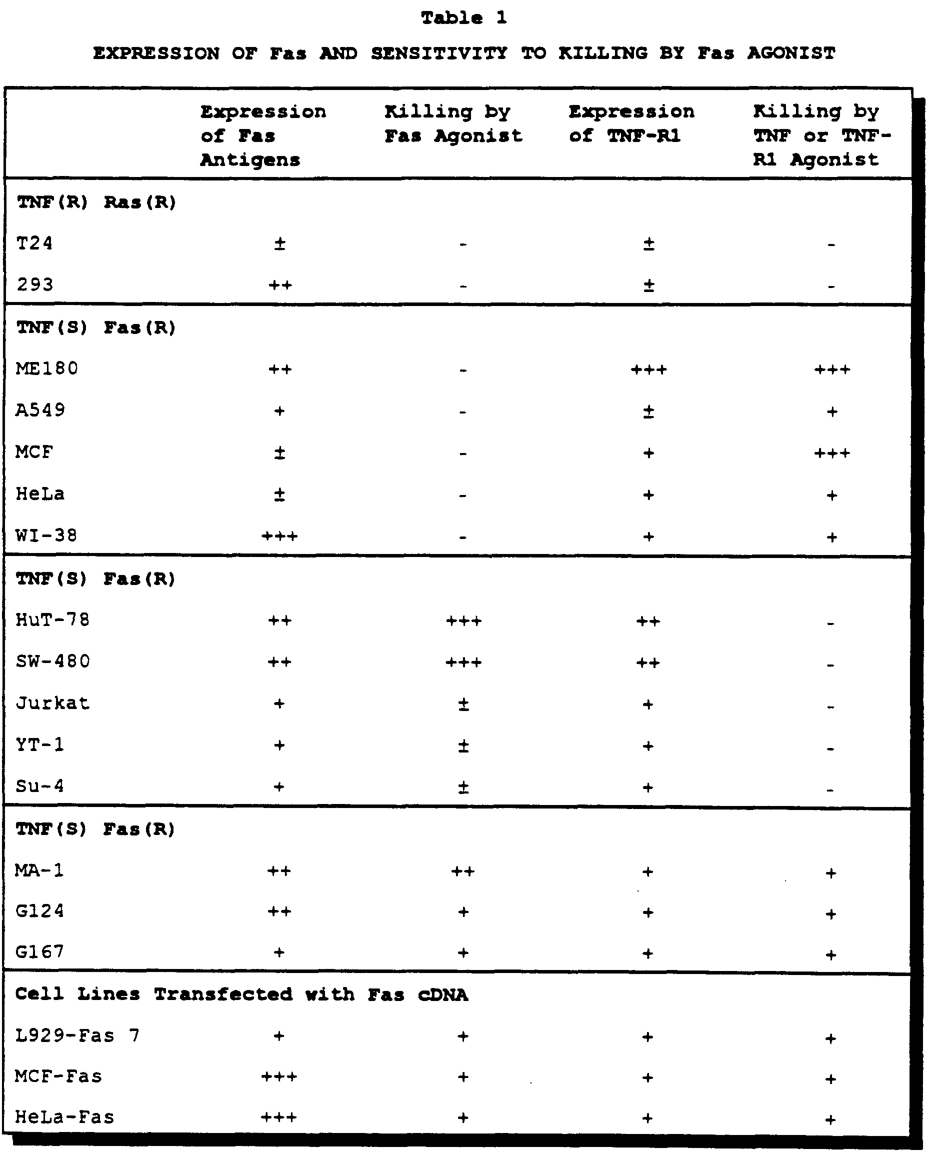

Human tumor cell lines such as ME180 (cervical carcinoma), A549 (lung fibroblast), MCF (breast carcinoma), HeLa (cervical carcinoma) and WI-38 (lung fibroblast), could be effectively killed by agonists of TNF-R1, but showed no sensitivity to Fas agonists (Table 1). Several of the Fas agonist resistant cell lines showed relatively low level expression of Fas receptor, which might explain their resistance. This was verified in the case of HeLa cells where, upon transfection with the human Fas cDNA, those cells expressing high levels of Fas antigen on their surface became sensitive to Fas agonists. However, this cannot explain the resistance of the ME-180 and WI-38 cell lines which both expressed high levels of Fas antigen as judged by flow cytometry. Therefore, although both ME-180 and WI-38 cells coexpress TNF-R1 and Fas antigen, they are selectively sensitive to only signals from TNF-R1.

The observations described above support a model in which Fas and TNF-R1 utilize distinct signaling pathways to mediate programmed cell death. Another test of distinct pathways leading to a common downstream effect is synergy between the two signals. Human bladder carcinoma T24 cells were found to be insensitive to both agonists of TNF-R1 and Fas (Fig. 4a). Less than 10% of the T24 cells were killed by TNF-R1 and Fas agonists when applied alone, even at the highest concentrations used. However, a combination of both Fas and TNF-R1 agonists killed the large majority of cells even at relatively low concentrations (Fig. 4). This striking synergy between TNF-R1 and Fas agonists strongly support the notion that Fas and TNF-R1 utilize distinct signal transduction pathways. Similar synergistic results were seen with cell lines that are somewhat sensitive to both anti-TNF-R1 and anti-Fas antibodies such as murine L929 cells expressing a transfected human Fas antigen cDNA (Fig. 4b) and the human glioblastoma A172 cell line (Fig. 4c). Synergy was also observed in SW-480, ME-180 and WI-38 cells (not shown), suggesting that this synergistic phenomenon is general and that the Fas antigen is able to signal in ME-190 and WI-38 cells.

CHI dramatically increases sensitivity of many cells to TNF killing probably through the inhibition of protective protein synthesis. Surprisingly, a 3 h pretreatment of MA-1 cells with CHI protects them from subsequent killing by treatment with TNF-α (Fig. 5a). In contrast, CHI pretreatment of MA-1 cells does not protect them from subsequent killing by Fas agonists.

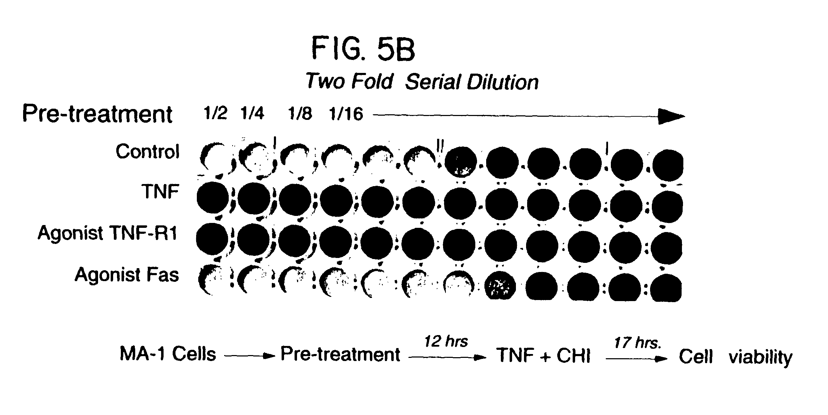

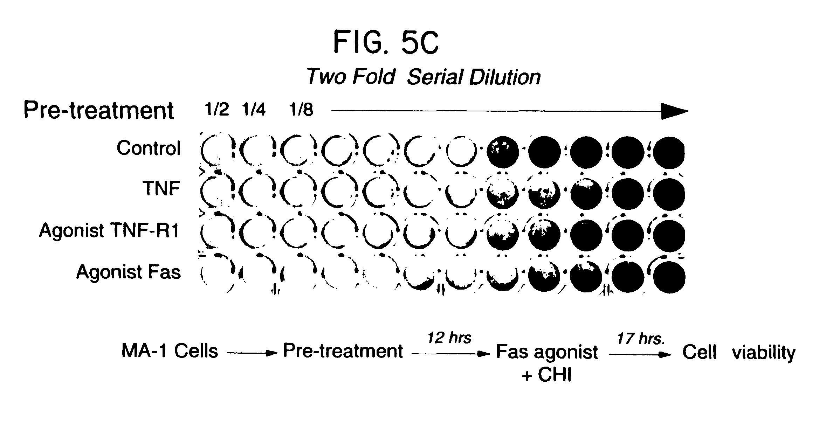

In addition to labile killing proteins, protective proteins such as MnSOD induced by TNF or IL-1 have been shown to protect cells against subsequent killing by TNF-α. We were therefore interested in whether TNF-a pretreatment would protect against killing by subsequent treatment with Fas antagonists + CHI. Fig. 5b shows that the TNF + CHI killing of MA-1 cells can be completely blocked by pretreatment with TNF-α. This induced resistance is not due to down regulation of TNF receptors and can be mimicked with IL-1. However, pretreatment of the MA-1 cells with TNF-α did not protect against subsequent killing by Fas agonists + CHI (Fig. 5c). In contrast, pretreatment of MA-1 cells with Fas agonists was unable to signal for a similar protective effect induced by TNF (Fig. 5b). These data provide additional evidence for distinct killing pathways mediated by TNF-R1 and Fas.

Fas antigen has been shown earlier to signal a programmed cell death indistinguishable from that mediated by TNF-R1, and it was suggested that homologous structures in their intracellular domains may mediate this signal. To determine if Fas can also signal these diverse activities, we examined the ability of both Fas and TNF-R1 to induce various cellular responses in cell lines coexpressing the two receptors. We found that Fas does not share TNF-Rl's ability to induce several biological activities known to be induced by TNF such as cell proliferation, anti-viral activity, and the induction of MnSOD, and ICAM. While TNF induces a multitude of cellular responses, signaling through Fas antigen seems to lead specifically to death by apoptosis of certain T-cells and tumor types. This highly specific action of anti-Fas antibodies suggests that agonists of Fas antigen (such as antibodies or the Fas ligand) may lead to effective anti-tumor therapies since they should not induce protection in the target tumors. Furthermore, an understanding of the distinct pathways of apoptosis mediated by TNF-R1 and Fas antigen may ultimately result in effective treatment of cancer or other diseases by the synergistic activities triggered through these two receptors.

Although the examples refer to particular preferred embodiments, it will be understood that the present invention is not so limited. It will occur to those ordinarily skilled in the art that various modifications may be made to the disclosed examples, without diverting from the overall concept of the invention.

The scope of the invention is defined by the appended claims.

## Claims

1. An in vitro method for cell lysis or inhibition of cell growth comprising treating cells expressing both the Fas antigen and the type 1 TNF receptor (TNF-R1) with an effective amount of a TNF-R1 agonist and a Fas agonist.

2. The method of claim 1 wherein said cells are selected from the group consisting of tumor cells, human immunodeficiency virus (HIV) infected cells and Epstein-Barr (EB) virus infected cells.

3. The method of claim 2 wherein said TNF-R1 agonist is TNF-α.

4. The method of claim 2 wherein said TNF-R1 agonist is an anti-TNF-R1 antibody.

5. The method of claim 2 wherein said Fas agonist is an anti-Fas antibody.

6. The method of claim 2 wherein said TNF-R1 agonist and said Fas agonist are used in a synergistically effective amount.

7. A (1) TNF-RI agonist and a Fas agonist for use in a method of medical treatment.

8. Use of a TNF-R1 agonist and a Fas agonist in preparation of a medicament for lysing or inhibiting the growth of cells expressing both the Fas antigen and the type 1 TNF receptor.

9. The use of claim 8 wherein the cells are those of a tumour bearing patient.

10. The use of claim 9 wherein the patient is human.

11. The method of claim 6 wherein said treatment is in the presence of cycloheximide (CH) or interferon gamma (IFN-γ).

12. A bispecific molecule comprising a first contiguous amino acid sequence specifically binding TNF-R1 and a second contiguous amino acid sequence specifically binding Fas antigen.

13. The bispecific molecule of claim 12 wherein said first amino acid sequence is selected from the group consisting of TNF-α, TNF-β and an agonist anti-TNF-R1 antibody.

14. The bispecific molecule of claim 12 wherein said second amino acid sequence is an agonist anti-Fas antibody.

15. The bispecific molecule of claim 12 further comprising an immunoglobulin sequence.

16. The bispecific molecule of claim 15 comprising a fusion of said first amino acid sequence to a first immunoglobulin constant domain sequence, and a fusion of said second amino acid sequence to a second immunoglobulin constant domain sequence.

17. The bispecific molecule of claim 16 wherein each of said first and second amino acid sequences is fused at its C-terminus to the N-terminus of an immunoglobulin heavy chain constant domain sequence comprising at least a hinge region and the C_{H}2 and C_{H}3 domains of an IgG-1, IgG-2 or IgG-3 immunoglobulin.

18. The bispecific molecule of claim 17 wherein said fusions are disulfide-linked.

19. The bispecific molecule of claim 18 wherein at least one of said fusions is associated with an immunoglobulin light chain.

20. A nucleotide sequence encoding the bispecific molecule of claim 12.

21. A replicable expression vector containing and capable of expressing, in a suitable host cell, the nucleotide sequence of claim 20.

22. A host cell transformed with the vector of claim 21.

23. A process comprising culturing the host cell of claim 22 so as to express the encoded bispecific molecule.

24. The process of claim 23 further comprising recovering the bispecific molecule from the host cell culture.

25. A pharmaceutical composition comprising a bispecific molecule of claim 12 in an amount capable of inducing cell lysis or inhibiting cell growth, in admixture with a pharmaceutically acceptable carrier.

26. An in vitro method for cell lysis or inhibition of cell growth comprising treating target cells carrying both the Fas antigen and the type 1 TNF receptor (TNF-R1) with an effective amount of the bispecific molecule of claim 12.

27. A synergistic composition of a TNF-R1 agonist and a Fas agonist.

## Patentansprüche

1. In vitro-Verfahren zur Zelllyse oder Hemmung von Zellwachstum, umfassend das Behandeln von Zellen, die sowohl das Fas-Antigen als auch den TNF-Rezeptor Typ 1 (TNF-R1) exprimieren, mit einer wirksamen Menge eines TNF-R1-Agonisten und eines Fas-Agonisten.

2. Verfahren nach Anspruch 1, worin die Zellen aus der aus Tumorzellen, mit Human-lmmundefizienzvirus (HIV) infizierten Zellen und mit Epstein-Barr- (EB-) Virus infizierten Zellen bestehenden Gruppe ausgewählt sind.

3. Verfahren nach Anspruch 2, worin der TNF-R1-Agonist TNF-α ist.

4. Verfahren nach Anspruch 2, worin der TNF-R1-Agonist ein Anti-TNF-R1-Antikörper ist.

5. Verfahren nach Anspruch 2, worin der Fas-Agonist ein Anti-Fas-Antikörper ist.

6. Verfahren nach Anspruch 2, worin der TNF-R1-Agonist und der Fas-Agonist in einer synergistisch wirksamen Menge eingesetzt werden.

7. Zusammensetzung, die einen TNF-R1-Agonisten und einen Fas-Agonisten umfasst, zur Verwendung in einem medizinischen Behandlungsverfahren.

8. Verwendung eines TNF-R1-Agonisten und eines Fas-Agonisten zur Herstellung eines Medikaments zur Lyse oder Hemmung des Wachstums von Zellen, die sowohl das Fas-Antigen als auch den TNF-Rezeptor Typ 1 exprimieren.

9. Verwendung nach Anspruch 8, worin die Zellen von einem Patienten mit einem Tumor stammen.

10. Verwendung nach Anspruch 9, worin der Patient ein Mensch ist.

11. Verwendung nach Anspruch 6, worin die Behandlung in Gegenwart von Cycloheximid (CH) oder Interferon-γ (IFN-γ) erfolgt.

12. Bispezifisches Molekül, das eine erste fortlaufende Aminosäuresequenz, die spezifisch TNF-R1 bindet, sowie eine zweite fortlaufende Aminosäuresequenz umfasst, die spezifisch Fas-Antigen bindet.

13. Bispezifisches Molekül nach Anspruch 12, worin die erste Aminosäuresequenz aus der aus TNF-α, TNF-β und einem Agonist-Anti-TNF-R1-Antikörper bestehenden Gruppe ausgewählt ist.

14. Bispezifisches Molekül nach Anspruch 12, worin die zweite Aminosäuresequenz ein Agonist-Anti-Fas-Antikörper ist.

15. Bispezifisches Molekül nach Anspruch 12, das weiters eine Immunglobulinsequenz umfasst.

16. Bispezifisches Molekül nach Anspruch 15, das eine Fusion aus der ersten Aminosäuresequenz an eine erste lmmunglobulin-Konstantdomänen-Sequenz sowie eine Fusion der zweiten Aminosäuresequenz an eine zweite lmmunglobulin-Konstantdomänen-Sequenz umfasst.

17. Bispezifisches Molekül nach Anspruch 16, worin jede von erster und zweiter Aminosäuresequenz an ihrem C-Terminus an den N-Terminus einer Immunglobulin-Schwerketten-Konstantdomänen-Sequenz fusioniert ist, die zumindest eine Hingeregion und die C_{H}2- und C_{H}3-Domänen eines lgG-1-, lgG-2 oder lgG-3-lmmunglobulins umfasst.

18. Bispezifisches Molekül nach Anspruch 17, worin die Fusionen Disulfid-verbunden sind.

19. Bispezifisches Molekül nach Anspruch 18, worin zumindest eine der Fusionen mit einer lmmunglobulin-Leichtkette assoziiert ist.

20. Nukleotidsequenz, die für das bispezifische Molekül nach Anspruch 12 kodiert.

21. Replizierbarer Expressionsvektor, der die Nukleotidsequenz nach Anspruch 20 enthält und in einer geeigneten Wirtszelle exprimieren kann.

22. Mit einem Vektor nach Anspruch 21 transformierte Wirtszelle.

23. Verfahren, umfassend das Kultivieren der Wirtszelle nach Anspruch 22, um das kodierte bispezifische Molekül zu exprimieren.

24. Verfahren nach Anspruch 23, weiters umfassend das Gewinnen des bispezifischen Moleküls aus der Wirtszellkultur.

25. Pharmazeutische Zusammensetzung, umfassend ein bispezifisches Molekül nach Anspruch 12 in einer Menge, die Zelllyse herbeiführen oder Zellwachstum hemmen kann, im Gemisch mit einem pharmazeutisch annehmbaren Träger.

26. In vitro-Verfahren zur Zelllyse oder Hemmung von Zellwachstum, umfassend die Behandlung von Zielzellen, die sowohl das Fas-Antigen als auch den TNF-Rezeptor Typ 1 (TNF-R1) tragen, mit einer wirksamen Menge des bispezifischen Moleküls nach Anspruch 12.

27. Synergistische Zusammensetzung aus einem TNF-R1-Agonisten und einem Fas-Agonisten.

## Revendications

1. Procédé in vitro pour la lyse cellulaire ou l'inhibition de la croissance cellulaire, comprenant le traitement de cellules exprimant à la fois l'antigène Fas et le récepteur de TNF de type 1 (TNF-R1) avec une quantité efficace d'un agoniste de TNF-R1 et d'un agoniste de Fas.

2. Procédé suivant la revendication 1, dans lequel les cellules sont choisies dans le groupe consistant en des cellules tumorales, des cellules infectées par le virus de l'immunodéficience humaine (VIH) et des cellules infectées par le virus d'Epstein-Barr (EB).

3. Procédé suivant la revendication 2, dans lequel l'agoniste de TNF-R1 consiste en TNF-α.

4. Procédé suivant la revendication 2, dans lequel l'agoniste de TNF-R1 est un anticorps anti-TNF-R1.

5. Procédé suivant la revendication 2, dans lequel l'agoniste de Fas est un anticorps anti-Fas.

6. Procédé suivant la revendication 2, dans lequel l'agoniste de TNF-R1 et l'agoniste de Fas sont utilisés en une quantité efficace de manière synergique.

7. Composition comprenant un agoniste de TNF-R1 et un agoniste de Fas, destinée à être utilisée dans une méthode de traitement médical.

8. Utilisation d'un agoniste de TNF-R1 et d'un agoniste de Fas dans la préparation d'un médicament destiné à lyser ou inhiber la croissance de cellules exprimant à la fois l'antigène Fas et le récepteur de TNF de type 1.

9. Utilisation suivant la revendication 8, dans laquelle les cellules sont des cellules d'un patient porteur d'une tumeur.

10. Utilisation suivant la revendication 9, dans laquelle le patient est un patient humain.

11. Procédé suivant la revendication 6, dans lequel le traitement est effectué en présence de cycloheximide (CH) ou d'interféron gamma (IFN-γ).

12. Molécule bispécifique comprenant une première séquence d'amino-acides contiguë se liant spécifiquement au TNF-R1 et une seconde séquence d'amino-acides contiguë se liant spécifiquement à l'antigène Fas.

13. Molécule bispécifique suivant la revendication 12, dans laquelle la première séquence d'amino-acides est choisie dans le groupe consistant en TNF-α, TNF-β et un anticorps agoniste anti-TNF-R1.

14. Molécule bispécifique suivant la revendication 12, dans laquelle la seconde séquence d'amino-acides est un anticorps agoniste anti-Fas.

15. Molécule bispécifique suivant la revendication 12, comprenant en outre une séquence d'immunoglobuline.

16. Molécule bispécifique suivant la revendication 15, comprenant une fusion de la première séquence d'amino-acides à une première séquence de domaine constant d'immunoglobuline, et une fusion de la seconde séquence d'amino-acides à une seconde séquence de domaine constant d'immunoglobuline.

17. Molécule bispécifique suivant la revendication 16, dans laquelle chacune des première et seconde séquences d'amino-acides est fusionnée à son extrémité C-terminale à l'extrémité N-terminale d'une séquence de domaine constant de chaîne lourde d'immunoglobuline comprenant au moins une région de charnière et les domaines C_{H}2 et C_{H}3 d'une immunoglobuline IgG-1, IgG-2 ou IgG-3.

18. Molécule bispécifique suivant la revendication 17, dans laquelle les fusions sont liées par des liaisons disulfures.

19. Molécule bispécifique suivant la revendication 18, dans laquelle au moins l'une des fusions est associée à une chaîne légère d'immunoglobuline.

20. Séquence de nucléotides codant pour la molécule bispécifique suivant la revendication 12.

21. Vecteur d'expression réplicable contenant et capable d'exprimer, dans une cellule-hôte convenable, la séquence de nucléotides suivant la revendication 20.

22. Cellule-hôte transformée avec le vecteur suivant la revendication 21.

23. Procédé comprenant la culture de la cellule-hôte suivant la revendication 22, de manière à exprimer la molécule bispécifique codée.

24. Procédé suivant la revendication 23, comprenant en outre l'étape consistant à recueillir la molécule bispécifique à partir de la culture de cellule-hôte.

25. Composition pharmaceutique comprenant une molécule bispécifique suivant la revendication 12, en une quantité capable d'induire une lyse cellulaire ou d'inhiber la croissance cellulaire, en mélange avec un support pharmaceutiquement acceptable.

26. Procédé in vitro pour la lyse cellulaire ou l'inhibition de la croissance cellulaire, comprenant le traitement de cellules cibles portant à la fois l'antigène Fas et le récepteur de TNF de type 1 (TNF-R1) avec une quantité efficace de la molécule bispécifique suivant la revendication 12.

27. Composition synergique d'un agoniste de TNF-R1 et d'un agoniste de Fas.
